# EUROPEAN PATENT APPLICATION

(11) **EP 4 822 703 A1**
(43) Date of publication of application: **14.10.2026**
(21) Application number: 24918080.3
(22) Date of filing: 06.09.2024
(51) Int. Cl.: G16B 40/20, G16B 5/00, G16H 50/20, G16H 50/30, G16H 70/20

(54) **DEVICE FOR IDENTIFICATION OR AUXILIARY IDENTIFICATION OF ACUTE CHEST PAIN TYPE AND USE THEREOF**

(30) Priority: 17.01.2024 CN 202410062876
(71) Applicant: BEIJING INSTITUTE OF HEART LUNG AND BLOOD VESSEL DISEASES, Beijing 100069 (CN)
(72) Inventor: DU, Jie, Beijing 100069 (CN); WANG, Yuan, Beijing 100069 (CN)
(74) Representative: Lorenz Seidler Gossel Part. mbB
(86) International application number: PCT/CN2024/117304
(87) International publication number: WO 2025/152471

(57) **Abstract**

Provided are a device for identification or auxiliary identification of an acute chest pain type and the use thereof in the field of medical care informatics. The technical problem to be solved is to quickly identify a chest pain type of a patient with acute chest pain. The device for identification or auxiliary identification of the acute chest pain type is provided, which device comprises the following modules: a data receiving module for receiving the content of a protein in the serum of a test subject, wherein the protein is soluble ST2, D-dieter and cardiac troponin I, and the test subject is a patient with acute chest pain; and a result output module for outputting the acute chest pain type from a computer on the basis of the concentration of the protein. The acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction. The provided device for identification or auxiliary identification of the acute chest pain type has a certain application prospect.

## Description

### Technical Field

The present invention relates to a device for identification or auxiliary identification of an acute chest pain type and use thereof in the field of medical care informatics.

### Background Art

Acute chest pain refers to non-traumatic pain or other chest discomfort that occurs within 24 hours of onset. Acute chest pain related to cardiovascular diseases includes acute myocardial infarction (AMI), acute aortic dissection (AAD), and acute pulmonary embolism (APE), which are extremely dangerous during onset and have a high mortality rate. They are all presented with symptoms of acute chest pain clinically, but different treatment methods are required because of different disease mechanisms. Acute myocardial infarction is caused by coronary thrombosis leading to myocardial ischemia. Acute pulmonary embolism is caused by the occlusion of pulmonary arteries by endogenous or exogenous emboli, resulting in pulmonary circulation and right heart dysfunctions. Both diseases require rapid diagnosis followed by anticoagulation, thrombolysis and other therapies. Acute aortic dissection means tearing of the intima and media of the aorta caused by various reasons, where the intima of the aorta is separated from the media, and the blood flows in, resulting in the aortic lumen being partitioned into a true lumen and a false lumen. If there is a diagnostic error, performing thrombolysis or other therapies may exacerbate the progression of a disease and endanger the patient's life. Therefore, there is an urgent need for biomarkers that can provide additional information beyond the existing clinical diagnostic methods to assist in the differential diagnosis of patients with acute fatal chest pain.

AMI, AAD and APE can all induce chest pain, thereby increasing the difficulty of clinical diagnosis and differential diagnosis, and leading to missed diagnosis and misdiagnosis. D-dimer is a specific degradation product produced by plasmin hydrolysis of cross-linked fibrin. When a detected value in the blood is below a positive threshold, it indicates that there is no thrombogenesis and thrombolysis in the body. AMI is caused by the occlusion of the coronary artery lumen by a thrombus. Since the arterial thrombus triggers platelet aggregation (white thrombus), the increase in D-dimer levels in the patient's body is less significant. In the case of AAD, the blood in the aortic lumen enters the middle layer of the aorta from transintimal tear, causing the media to be separated to form a thrombus, so the level of D-dimer in the body generally increases. Once a thrombus is formed in the pulmonary artery of a patient with acute pulmonary embolism, it will stimulate a fibrinolytic system of an organism. As a result, the level of D-dimer in the patient's body will significantly increase within a short time. A D-dimer biomarker shows an increase in both acute aortic dissection and acute pulmonary embolism.

ST2 is a receptor for interleukin 33 (IL-33), including a soluble ST2 receptor (sST2) and a membrane-bound functional receptor (ST2L). The preliminary research results show that among patients with aortic dissection, compared with those with acute myocardial infarction, the sST2 level is higher in patients with acute aortic dissection within 24 hours of symptom onset; and compared with those with acute pulmonary embolism, the sST2 level is even higher in patients with acute aortic dissection, indicating that its diagnostic value is quite significant and can be used as an auxiliary indicator for aortic dissection.

Troponin (Tn), as a regulatory protein for muscle tissue contraction, is located on a thin filament of a contractile protein, and plays an important regulatory role in muscle contraction and relaxation. It contains three subtypes: a fast-reactive type, a slow-reactive type, and cardiac troponin (cTn). The former two are related to skeletal muscles, while cardiac troponin is only present in cardiomyocytes and is a complex composed of three subunits: cardiac troponin T (cTnT), cardiac troponin I (cTnI), and cardiac troponin C (cTnC). cTnT and cTnI are antigens specific to cardiomyocytes. cTnI has a molecular weight of about 23.88 KDa and is degraded from myocardial fibers when cardiomyocytes are injured. The increase of cTn in serum reflects an injury of cardiomyocytes. Its specificity and sensitivity are both higher than those of the previously commonly used myocardial enzyme spectrum. During myocardial infarction, due to occlusion or near occlusion of blood vessels, severe myocardial ischemia occurs, which in turn leads to the necrosis of cardiomyocytes. cTnI within cells is released into the blood, causing an increase of cTnI in the serum, which is a marker for the destruction of a myocardial structure and reflects the pathologically qualitative diagnosis.

Currently in clinical practice, the differential diagnosis of patients with acute fatal chest pain mainly relies biomarkers known by patients, imaging findings, and pathogenesis characteristics, etc. Only D-dimer has clinical significance in the diagnosis of suspected patients with AAD. However, it can only be used for differential diagnosis of patients with acute myocardial infarction, but cannot distinguish patients with AAD from patients with pulmonary embolism. In addition, a large-scale instrument for detection needs to be used in a laboratory in the detection process. The entire detection process takes approximately 4 hours or more. The detection of other metabolites also needs to be carried out in the laboratory and is even more time-consuming. In addition, individual biomarkers can only reflect part of disease information from a single perspective and cannot comprehensively and accurately assess the causes, as well as pathological and pathophysiological changes of acute chest pain and possibly accompanied dyspnea. Therefore, there is an urgent need for biomarkers that can provide additional information beyond the existing clinical diagnosis methods to assist in the differential diagnosis of patients with chest pain. In response to the urgent need for rapid differential diagnosis of population with acute chest pain, a new strategy for rapid differential diagnosis of acute fatal chest pain has been established, and a kit suitable for rapid diagnosis has been developed.

### Summary of the Invention

### Technical problem

The technical problem to be solved by the present invention is how to quickly identify a chest pain type of a patient with acute chest pain, in order to provide an effective treatment strategy.

### Technical solution

To solve the aforementioned technical problem, the present invention provides a device for identifying or assisting in identifying an acute chest pain type.

The device for identifying or assisting in identifying the acute chest pain type provided by the present invention comprises the following modules:
M1, a data receiving module configured to receive a content of a protein in a serum of a test subject, wherein the protein is selected from the group consisting of soluble ST2, D-dimer and cardiac troponin I, and the test subject is a patient with acute chest pain; and
M2, a result output module configured to output, from a computer, the acute chest pain type on the basis of a concentration of the protein.

The acute chest pain type may be acute aortic dissection, acute myocardial infarction, acute pulmonary embolism or other type of acute chest pain, wherein the other type of acute chest pain may be acute chest pain other than acute aortic dissection, acute myocardial infarction and acute myocardial infarction.

The soluble ST2 may be a protein having an amino acid sequence as shown by SEQ ID NO: 1 in a sequence listing. The cardiac troponin I may be a protein having an amino acid sequence as shown by SEQ ID NO: 2 in the sequence listing.

In the aforementioned device, the result output module comprises a model construction sub-module; and the model construction sub-module is used to construct a model by taking the concentration of the protein in the serum of a patient with acute chest pain as input data of the model and taking the acute chest pain type as output data.

The aforementioned device may be a computer device and comprises a memory, a processor, and a computer program that is stored in the memory.

The memory is used to store a program therein. Specifically, the program may comprise program codes, which comprise computer program instructions.

The memory may comprise an internal storage and a non-volatile memory, and provide instructions and data to the processor.

The processor is used to read the corresponding computer program from the non-volatile memory into the internal storage and then run said program, forming a screening device for acute chest pain types at a logical level.

The processor is used to execute the computer program to implement the following steps:
N1, receiving data: receiving a content of a protein in the serum of a test subject, wherein the protein is selected from the group consisting of soluble ST2, D-dimer and cardiac troponin I, and the test subject is a patient with acute chest pain; and
N2, outputting a result: outputting an acute chest pain type from a computer on the basis of a concentration of the protein.

The acute chest pain type may be acute aortic dissection, acute myocardial infarction, acute pulmonary embolism or other type of acute chest pain, wherein the other type of acute chest pain may be acute chest pain other than acute aortic dissection, acute myocardial infarction and acute myocardial infarction.

These computer program instructions may further be stored in a computer-readable memory that can guide a computer or other programmable data processing devices to work in a specific way, such that manufactured products including an instruction device may be created by the instructions stored in this computer-readable memory. This instruction device implements the functions designated in one or more processes in the flowcharts and/or in one or more blocks in the block diagrams.

The computer program instructions may also be loaded onto the computer or other programmable data processing devices, such that a series of operation steps are executed on the computer or other programmable devices to produce computer-implemented processing. Therefore, the instructions executed on the computer or other programmable devices provide steps for implementing the functions designated in one or more processes in the flowcharts and/or in one or more blocks in the block diagrams.

The present invention further provides a method for constructing a prediction model for an acute chest pain type. The method comprises: receiving a content of a protein in a serum of a patient with acute chest pain, wherein the protein is selected from the group consisting of soluble ST2, D-dimer and cardiac troponin I; and constructing a prediction model for an acute chest pain type by taking the content of the protein as model input data and taking the acute chest pain type as output data.

The present invention further provides a method for identifying or assisting in identifying an acute chest pain type. The method comprises: receiving a content of a protein in the serum of a patient with acute chest pain, wherein the protein is selected from the group consisting of soluble ST2, D-dimer and cardiac troponin I; and outputting the acute chest pain type from a computer on the basis of a concentration of the protein, wherein the acute chest pain type is acute aortic dissection, acute myocardial infarction, acute pulmonary embolism or other type of acute chest pain, and the other type of acute chest pain is acute chest pain other than acute aortic dissection, acute myocardial infarction and acute myocardial infarction.

In the aforementioned device, the method further comprises a step of constructing a model. The constructing the model comprises: receiving the content of soluble ST2, D-dimer and cardiac troponin I in the serum of a patient with acute chest pain as model input data; and constructing the prediction model for the acute chest pain type by means of using the model input data and by taking the acute chest pain type as output data.

The present invention further provides a computer program product or a computer-readable storage medium. The computer program product comprises a computer program. The computer-readable storage medium is used to store a computer program, wherein the computer program is executed by a processor to implement the steps of the aforementioned method for identifying or assisting in identifying an acute chest pain type.

The computer program product may be a software product that mainly implements its solutions through the computer program.

The computer-readable storage medium refers to a carrier for storing data and comprises both persistent and non-persistent and removable and non-removable media that may be implemented by any method or technology for storage of information. The information may be computer-readable instructions, data structures, program modules or other data. The computer-readable storage medium may be a magnetic tape, a magnetic disk, a floppy disk, an optical disk, a magneto-optical disk, ROM, PROM, VCD, DVD, a hard disk, a flash memory, a U disk, a CF card, an SD card, an MMC card, an SM card, a memory stick, or an xD card, etc.

The present invention further provides a product for identifying or assisting in identifying an acute chest pain type. Wherein the product comprises a device and a reagent, the device is the aforementioned device; the reagent is a protein and/or a substance for the detection of the protein; the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

The substance may comprise a substance for the detection of the content of a protein in the serum by means of enzyme-linked immunosorbent assay, immunofluorescence assay, flow cytometry, radioimmunoassay, immunocoprecipitation, immunoblotting, high-performance liquid chromatography, capillary gel electrophoresis, near-infrared spectroscopy, mass spectrometry, immunochemiluminescence, a colloidal gold immunoassay technology, a fluorescence immunochromatography technology, a surface plasmon resonance technology, an immuno-PCR technology, or a biotin-avidin technology.

The product may be a reagent and/or an instrument. The product may be a system.

The present invention further provides use of a protein in the preparation of a product for identifying or assisting in identifying an acute chest pain type, wherein the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

Use of a substance for the detection of the aforementioned protein in the preparation of a product for identifying or assisting in identifying an acute chest pain type also falls within the protection scope of the present invention.

In the aforementioned use, the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

The aforementioned use or method may be use or a method for non-disease diagnosis. The aforementioned use or method is not directly intended to obtain a disease diagnosis result or health status in a living human or animal body.

The aforementioned use or method may be use or a method for disease diagnosis.

The aforementioned use or method may be use or a method for a non-disease treatment purpose. The aforementioned use or method is not intended to restore or gain health or reduce pain in a living human or animal body.

The aforementioned use or method may be use or a method for a disease treatment purpose.

### Beneficial effects

According to the present invention, the product for differential diagnosis or assisting in differential diagnosis of a patient with acute chest pain can be prepared by combination of soluble ST2, D-dimer and cardiac troponin I in the serum as indicators for the differential diagnosis of acute chest pain. The differential diagnosis of a patient with acute chest pain can be performed by detecting soluble ST2, D-dimer and cardiac troponin I in the serum. The present invention can improve the ability of differential diagnosis for acute chest pain based on known biomarkers, and is used in clinical practice to precisely stratify patient risks, thereby increasing survival rates of patients.

### Brief Description of the Drawings

FIG. 1 is a computer flowchart of a method for identifying or assisting in identifying an acute chest pain type in the present invention.
FIG. 2 shows the content of three markers for patients in a discovery queue. A represents an sST2 level in the serum; B represents a D-dimer level in the serum; and C represents the comparison of cardiac troponin levels in the serum. The ordinate represents the sST2 level in the serum, the D-dimer level in the serum, and the cardiac troponin I level in the serum, respectively, in ng/mL.

### Detailed Description of the Invention

The present invention is described in further detail below in conjunction with the specific embodiments, and the given examples are only for the purpose of clarifying the present invention, but not for the purpose of limiting the scope of the present invention. The examples provided below may be used as a guide for further improvement by a person of ordinary skill in the art and do not in any way constitute a limitation of the present invention.

The experimental methods in the following examples, unless otherwise specified, are conventional methods and are carried out in accordance with the techniques or conditions described in the literatures in the art or in accordance with the product instructions. The materials, reagents, etc. used in the following examples, unless otherwise specified, may be obtained commercially.

A serum soluble ST2 detection kit Human ST2/IL-33R in the following examples was purchased from R&D Corporation, with Catalog No. DY523B-05. An ACL TOP550CTS fully-automatic coagulation analyzer was used for D-dimer detection. A Beckman fully-automatic luminescence analyzer was used for cardiac troponin I. (The term "cardiac troponin" as mentioned below refers to "cardiac troponin I")

Stata/SE 15.1 statistical software was used in the following examples to process data. An experimental result of normally distributed continuous variables was expressed as mean ± standard deviation by using One-way ANOVA test. An experimental result of non-normally distributed continuous variables was expressed as a median (25%-75%) by using rank sum test. An experimental result of categorical variables was expressed by chi-square test. P<0.05(*) indicated a significant difference, P<0.01(**) indicated a very significant difference, and P<0.001(***) indicated a very significant difference.

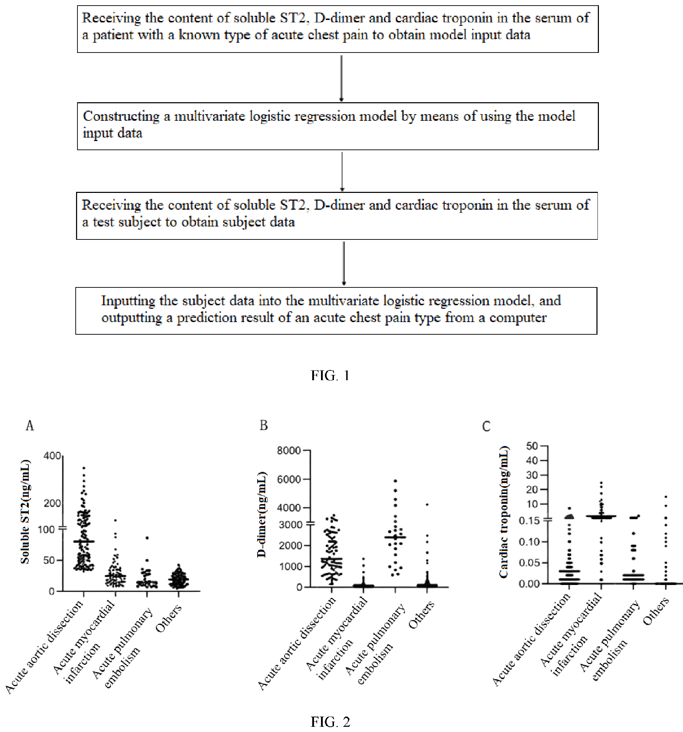

The present invention first provides a method for identifying or assisting in identifying an acute chest pain type (see FIG. 1). The method comprises the following steps:
Step S1: receiving the content of soluble ST2, D-dimer and cardiac troponin in the serum of a patient with a known type of acute chest pain to obtain model input data;
Step S2: constructing a multivariate logistic regression model by means of using the model input data;
Step S3: receiving the content of soluble ST2, D-dimer and cardiac troponin in the serum of a test subject to obtain subject data; and
Step S4: inputting the subject data into the multivariate logistic regression model, and outputting a prediction result of an acute chest pain type from a computer.
Example 1: Combined differential diagnosis of chest pain types by soluble ST2 (sST2), D-dimer and cardiac troponin

### Ethical statement

An experimental protocol in this example of the present invention was approved by the Ethics Committee of Beijing Anzhen Hospital, Capital Medical University. The research was conducted in accordance with the principles of "Declaration of Helsinki".

### Research objects

All the research objects were adult individuals from China.

Diagnostic criteria for patients

The patients incorporated in the research needed to meet the following conditions.

Acute chest pain: the latest management consensus released by the European Society of Cardiology (ESC) and the Acute Cardiovascular Care Association (ACCA) in 2020 clearly stated that acute chest pain refers to non-traumatic pain or other chest discomfort that occurs within 24 hours of onset.

Acute myocardial infarction (abbreviated as AMI) means acute myocardial injury [an increase or decrease of cardiac troponin (cTn) in the serum at least once exceeds an upper limit (a 99th percentile value of an upper limit of a reference range) of a normal value], along with clinical evidences of acute myocardial ischemia, including: (1) acute myocardial ischema symptoms; (2) new ischemic electrocardiogram changes; (3) new pathological Q waves; (4) new imaging evidences of loss of viable myocardium or segmental movement abnormalities of the ventricular wall; and (5) coronary artery thrombosis confirmed by coronary angiography or intraluminal imaging examination or autopsy.

Acute aortic dissection (abbreviated as AAD) means tearing of the intima and media of the aorta caused by various reasons, where the intima of the aorta is separated from the media, and the blood flows in, resulting in the aortic lumen being partitioned into a true lumen and a false lumen. The onset time of less than or equal to 2 weeks means an acute phase.

Acute pulmonary embolism (abbreviated as APE) is a clinical syndrome caused by the occlusion of pulmonary arteries by endogenous or exogenous emboli, resulting in pulmonary circulation and right heart dysfunctions.

The other type of acute chest pain suffered by the patient is acute chest pain other than acute aortic dissection, acute myocardial infarction and acute myocardial infarction, including unstable angina, chest pain symptoms or other diseases. Diagnostic criteria for unstable angina: a group of clinical angina syndromes between stable angina and acute myocardial infarction.

Discovery queue: a total of 326 patients with acute chest pain were included, wherein an average age of 326 patients was 53 years old, and 219 males were included, accounting for 68.4%. The baseline characteristics of this discovery queue were stratified by disease types, as detailed in Table 1. Among 326 patients, there were 109 patients with AAD, 72 patients with AMI, 24 patients with APE, and 121 patients with other type of acute chest pain. Among 109 patients with AD, the average age was 51 years old, 68.9% of the patients were male, the median body mass index (BMI) was 26.6, the median left ventricular ejection fraction (LVEF) was 63.0%, and 69.4% of the patients had high blood pressure (HBP), while 3.5% had diabetes mellitus (DM). Among 72 patients with AMI, the average age was 54 years old, 84.7% of the patients were male, the median BMI was 25.8, the median LVEF was 55.0%, and 59.4% of the patients had high blood pressure, while 26.1% had diabetes mellitus. Among 24 patients with acute pulmonary embolism (APE), the average age was 62 years old, 50% of the patients were male, the median BMI was 23.8, the median LVEF was 62.0%, and 58.3% of the patients had high blood pressure, while 20.8% had diabetes mellitus. There were 121 patients with other type of acute chest pain, an average age was 53 years old, and 62.0% of the patients were male.

**Table 1: Statistical information on patients with acute chest pain in a discovery queue**

| | 326 patients with acute chest pain | 109 patients with AAD | 72 patients with AMI | 24 patients with APE | 121 patients with other type of acute chest pain | P |
|---|---|---|---|---|---|---|
| Age(years) | 53.1±11.9 | 50.8±11.7 | 53.8±11.4 | 62.4±9.6 | 52.7±12.0 | <0.00 1 |
| Male(%) | 219(68.4) | 71(68.9) | 61(84.7) | 12(50.0) | 75(62.0) | 0.002 |
| BMI(Kg/M²) | 26.3(24.6-29.3 ) | 26.6(24.5-28.7) | 25.8 (22.7-29.4) | 23.8(23.7-29.3) | 27.1(25.7-29.4) | 0.487 |
| LVEF | 60.0 (55.0-65.0) | 63.0(60.0-68.0) | 55.0(45.0-60.0) | 62.0(60.0-66.0) | 67.5(62.5-68.0) | <0.00 1 |
| HBP | 131(63.0) | 59(69.4) | 41(59.4) | 14(58.3) | 17(56.7) | 0.454 |
| DM | 33(15.8) | 3(3.5) | 18 (26.1) | 5 (20.8) | 7(23.3) | 0.001 |
| gST2 concentration(ng/m L) | 29.2(15.9-54.5 ) | 80.0(49.2-143.8) | 25.0(l5.5-37.2) | 14.9(10.2-30.1) | 19.1(11.8-26.1) | <0.00 1 |
| D-dimer concentration(ng/m L) | 156.0 (61.0-1073.0) | 1356.0(866.0-2406. 5) | 71.5(52.5-126. 0) | 2393.5(1373.0-2990. 5) | 75.0(45.0-186. 0) | <0.00 1 |
| Cardiac troponin concentration(ng/m L) | 0.02(0-0.18) | 0.03(0.01-0.08) | 0.66(0-13-3.88) | 0.02(0.01-0.09) | 0(0-0.01) | <0.00 1 |

Discovery queue: a total of 217 patients with acute chest pain were included, wherein an average age of 217 patients was 53 years old, and 163 males were included, accounting for 75.1%. The baseline characteristics of this discovery queue were stratified by disease types, as detailed in Table 2. Among 217 patients, there were 120 patients with AAD, 70 patients with AMI, and 27 patients with APE. Among 120 AD patients, the average age was 48 years old, 77.5% of the patients were male, the median BMI was 26.1, the median LVEF was 63.0%, and 76.7% of the patients had high blood pressure, while 8.3% had diabetes mellitus. Among 70 patients with AMI, the average age was 57 years old, 85.7% of the patients were male, and 58.6% of the patients had high blood pressure, while 28.6% had diabetes mellitus. Among 27 patients with APE, the average age was 66 years old, 37.0% of the patients were male, and 59.3% of the patients had high blood pressure, while 14.8% had diabetes mellitus.

**Table 2: Statistical information on patients with acute chest pain in a validation queue**

| | 217 patients with acute chest pain | 120 patients with AAD | 70 patients with AMI | 27 patients with APE | P |
|---|---|---|---|---|---|
| Age(years) | 53.1±13.0 | 47.8±11.2 | 57.1±10.3 | 65.7±14.5 | <0.001 |
| Male(%) | 163(75.1) | 93(77.5) | 60(85.7) | 10(37.0) | <0.001 |
| HBP | 149(68.7) | 92(76.7) | 41(58.6) | 16(59.3) | 0.018 |
| DM | 34(15.7) | 10(8.3) | 20(28.6) | 4(14.8) | 0.001 |
| sST2 concentration(ng/mL) | 32.6(15.2-85.9) | 72.5(39.2-138.1) | 15.4(10.8-25.3) | 10.7(7.4-19.1) | <0.001 |
| D-dimer concentration(ng/mL) | 963.0(131.0-1992.0) | 1470.5(944.5-2651.5) | 91.5(57.0-131.0) | 1259.0(937.0-2318.0) | <0.001 |
| Cardiac troponin concentration(ng/mL) | 0.07(0.01-0.85) | 0.02(0.01-0.34) | 0.67(0.11-2.45) | 0.04(0.01-0.19) | <0.001 |

I. Detection of the content of soluble ST2, D-dimer and cardiac troponin in the serum Serum sample processing: the blood was collected and centrifuged at 4°C and 1200 rmp for 10 minutes, and the centrifuged supernatant was collected for testing.

### 1. Detection of soluble ST2 in the serum

Detection kit: Human ST2/IL-33R DuoSet (R&D, Catalog No. DY523B-05).The detection steps for serum sST2 by an ELISA method were carried out with reference to instructions in the kit.

### 2. Detection of D-dimer in the serum

Detection kit: Catalog No. REV202209, Instrument Laboratory Company. The ACL TOP550CTS fully-automatic coagulation analyzer was used, and the detection steps for D-dimer levels in the serum were carried out in accordance with instructions in the kit.

### 3. Detection of cardiac troponin I in the serum

Detection kit: Catalog No. B52699, manufactured by Immunotech SAS. The Beckman fully-automatic luminescence analyzer was used, and the detection steps for the cardiac troponin level in the serum were carried out in accordance with instructions in the kit.

### II. Analysis of data results of individual markers for soluble ST2, D-dimer and cardiac troponin levels of samples in a discovery queue

The results were shown in FIG. 2 (A, B and C) and Table 1: compared with other type of chest pain (other than AAD), the sST2 level in the serum of a patient with AAD was significantly increased, being 80.0 (median, IQR 49.2-143.8) (P<0.001).

Compared with other type of chest pain (other than APE), the D-dimer level in the serum of a patient with APE was significantly increased, being 2394 (median, IQR 1373-2991) (P<0.001). Compared with other type of chest pain (other than AMI), the cTNI level in the serum of a patient with AMI was significantly increased, being 0.66 (median, IQR 0.13-3.88) (P<0.001).

### III. Combined differential diagnosis of different types of chest pain by three markers - soluble ST2, D-dimer and cardiac troponin

To achieve the optimal combination of three biomarkers - soluble ST2, D-dimer and cardiac troponin, a multivariate logistic regression model was constructed using different variable combinations (random combinations of the three biomarkers - soluble ST2, D-dimer, and/or cardiac troponin). A data processing and analysis method was as follows: data was processed using Stata/SE 15.1 statistical software. In the presence of multiple predictive variables, the multivariate logistic regression model was adopted. AUC was calculated based on a prediction probability generated by the multivariate logistic regression model. A cutoff value for the combined diagnosis by ST2, D-dimer and cardiac troponin was derived as an optimal threshold through calculation of a Youden index (Youden index = sensitivity + specificity - 1).

The results were as follows: the combination of soluble ST2, D-dimer and cardiac troponin performed best in terms of diagnostic performance; in the aforementioned discovery queue, AUC (0.9813) in combined diagnosis of ADD by soluble ST2, D-dimer and cardiac troponin was higher than that in respective diagnosis by a single marker, with a sensitivity of 98.06% and a specificity of 90.19%; AUC (0.9054) in combined diagnosis of AMI by soluble ST2, D-dimer and cardiac troponin was higher than that in respective diagnosis by a single marker, with a sensitivity of 76.39% and a specificity of 91.43%; and AUC (0.9554) in combined diagnosis of APE by soluble ST2, D-dimer and cardiac troponin was higher than that in respective diagnosis by a single marker, with a sensitivity of 91.30% and a specificity of 90.11% (Table 3).

**Table 3: ROC curve information of patients with various types of acute chest pain in individual or combined diagnosis by sST2, D-Dimer and cTNI in a discovery queue**

| Acute chest pain type | Markers | AUC | Sensitivity | Specificity | Threshold(cutoff) |
|---|---|---|---|---|---|
| AAD versus other type of chest pain | sST2 | 0.9663 | 99.03% | 84.79% | 34.58 |
| | D-Dimer | 0.8888 | 97.22% | 78.80% | 324 |
| | cTNI | 0.5082 | 80.58% | 39.72% | 0.01 |
| | sST2+D-Dimer+cTNI | 0.9813 | 98.06% | 90.19% | 0.1609 |
| AMI versus other type of chest pain | sST2 | 0.4175 | 100.00% | 8.06% | 8.93 |
| | D-Dimer | 0.2469 | 97.22% | 2.82% | 27 |
| | cINI | 0.8985 | 93.06% | 70.20% | 0.04 |
| | sST2+D-Dimer+cTNI | 0.9054 | 76.39% | 91.43% | 0.3139 |
| APE versus other type of chest pain | sST2 | 0.3025 | 100.00% | 3.04% | 6.94 |
| | D-Dimer | 0.8981 | 100.00% | 9.81% | 495 |
| | cTNI | 0.5338 | 91.30% | 35.03% | 0.01 |
| | sST2+ D-Dimer+cTNI | 0.9554 | 91.30% | 90.11% | 0.0725 |

### IV. Classification of different types of acute chest pain by combination of biomarkers - soluble ST2, D-dimer and cardiac troponin

It was found by combined diagnosis of markers in the discovery queue - soluble ST2, D-dimer and cardiac troponin (Table 4): when the content of sST2 was ≥ 34.6 ng/mL, a diagnostic rate of acute aortic dissection was 92.7%; when the content of cTNI was ≥ 0.04 ng/mL, a diagnosis rate of acute myocardial infarction was 93.1%; and when the content of sST2 was < 34.6 ng/mL and the content of D-Dimer was ≥ 500 ng/mL, a diagnostic rate of acute pulmonary embolism was 87.5%.

**Table 4: Statistical data on the classification of different types of chest pain by markers in a discovery queue**

| Different types of chest pain were classified according to sST2&D-Dimer&cTNI levels by combination of markers | 109 patients with AAD | 72 patients with AMI | 24 patients with APE |
|---|---|---|---|
| sST2≥34.6ng/mL, D-Dimes-, cTNI- | 101(92.7%) | 23(31.9%) | 3(12.5%) |
| sST2-, D-Dimer-, cTNI≥0.04ng/mL | 42(38.5%) | 67(93.1%) | 0 |
| sST2<34.6 ng/mL, D-Dimer≥500ng/mL, cTNI- | 1(0.9%) | 1(1.4%) | 21(87.5%) |

In summary, the prediction model for different types of acute chest pain was constructed by combination of soluble ST2, D-dimer and cardiac troponin in the follow ways:
1) when the content of sST2 was ≥ 34.6 ng/mL, it assists in identifying the chest pain type as acute aortic dissection;
2) when the content of cTNI was ≥ 0.04 ng/mL, it assists in identifying the chest pain as acute myocardial infarction;
3) when the content of sST2 was < 34.6 ng/mL and the content of D-Dimer was ≥ 500 ng/mL, it assists in identifying the chest pain type as acute pulmonary embolism; and
4) when the content of sST2 was < 34.6 ng/mL, the content of D-Dimer was < 500 ng/mL, and the content of TNI was < 0.04 ng/mL, it assists in identifying the chest pain type as other type of chest pain other than the above three types of acute fatal chest pain.

This prediction model was then validated in a validation queue.

Example 2: Use of combined soluble ST2, D-dimer and cardiac troponin in the prediction model of chest pain type

The content of soluble ST2, D-dimer and cardiac troponin in the serum of samples in the validation queue were detected according to the detection method described in Example 1 (see

Table 7 for specific data), and then the prediction model in Example 1 was used to perform multivariate logistic regression on the validation queue.

The results were as follows: in Table 5, sST2 represented the diagnosis by sST2 alone, and sST2+D-Dimer+cTN1 represented combined diagnosis by these three proteins - soluble ST2, D-dimer and cardiac troponin. In the validation queue, AUC (0.9534) in combined diagnosis of ADD by these three proteins - soluble ST2, D-dimer and cardiac troponin was higher than that in respective diagnosis by a single marker, with a sensitivity of 93.33% and a specificity of 84.54%; AUC (0.9849) in combined diagnosis of these three proteins - AMI by soluble ST2, D-dimer and cardiac troponin was higher than that in respective diagnosis by a single marker, with a sensitivity of 97.14% and a specificity of 96.60%; and AUC (0.9076) in combined diagnosis of APE by soluble ST2, D-dimer and cardiac troponin was higher than that in respective diagnosis by a single marker, with a sensitivity of 92.59% and a specificity of 86.84% (Table 5).

**Table 5: ROC curve information of patients with various types of acute chest pain in individual or combined diagnosis by sST2, D-Dimer and cTNI in a validation queue**

| Acute chest pain type | Markers | AUC | Sensitivity | Specificity | Threshold(cutoff) |
|---|---|---|---|---|---|
| AAD versus other type of chest pain | sST2 | 0.9302 | 82.50% | 92.78% | 34.73% |
| | D-Dimer | 0.8446 | 95.00% | 69.07% | 391 |
| | cTNI | 0.3061 | 100.00% | 0.00% | 0 |
| | sST2+D-Dimer+cTNI | 0.9534 | 93.33% | 84.54% | 0.3889 |
| AMI versus other type of chest pain | sST2 | 0.1765 | 100.00% | 0.00% | 4.60 |
| | D-Dimer | 0.0267 | 100.00% | 0.00% | 11 |
| | cTNI | 0.7730 | 88.57% | 54.42% | 0.04 |
| | sST2+D-Dimer+cTNI | 0.9849 | 97.14% | 96.60% | 0.3690 |
| APE versus other type of chest pain | sST2 | 0.1728 | 100.00% | 0.53% | 5.22 |
| | D-Dimer | 0.6673 | 88.89% | 41.58% | 500 |
| | cTNI | 0.3924 | 81.48% | 16.32% | 0.01 |
| | sST2+D-Dimer+cTNI | 0.9076 | 92.59% | 86.84% | 0.1838 |

When the content of sST2 was ≥ 34.6 ng/mL, the chest pain was diagnosed as acute aortic dissection; when the content of cTNI was ≥ 0.04 ng/mL, the chest pain was diagnosed as acute myocardial infarction; and when the content of sST2 was < 34.6 ng/mL and the content of D-Dimer was ≥ 500 ng/mL, the chest pain was diagnosed as acute pulmonary embolism.

It was found by combined diagnosis of markers in the validation queue - soluble ST2, D-dimer, and cardiac troponin (Table 6 and Table 7): when the content of sST2 was ≥ 34.6 ng/mL, a diagnostic rate of acute aortic dissection was 82.50%; when the content of cTNI was ≥ 0.04 ng/mL, a diagnosis rate of acute myocardial infarction was 88.57%; and when the content of sST2 was < 34.6 ng/mL and the content of D-Dimer was ≥ 500 ng/mL, a diagnostic rate of acute pulmonary embolism was 85.19%.

**Table 6: Statistical data on the classification of different types of chest pain by combination of markers in a validation queue**

| Different types of chest pain were classified according to sST2&D-Dimer&cTNI levels by combination of markers | 120 patients with AAD | 70 patients with AMI | 27 patients with APE |
|---|---|---|---|
| sST2≥34.6ng/mL, D-Dimer-, cTNI- | 99(82.50%) | 6(8.57%) | 1(3.70%) |
| sST2-, D-Dimer-, cTNI≥0.04ng/mL | 52(43.33%) | 62(88.57%) | 15(55.56%) |
| sST2<34.6 ng/mL, D-Dimer≥500 ng/mL, cTNI- | 20(16.67%) | 3(4.29%) | 23(85.19%) |

**Table 7. Detection results of soluble ST2, D-dimer and cardiac troponin levels in the serum of patients in a validation queue**

| Patent Nos. | Soluble ST2 concentration(ng/mL) | D-dimer concentration(ng/mL) | Cardiac troponin concentration(ng/mL) | Determination results of chest pain types |
|---|---|---|---|---|
| 1 | 8.13613779 | 999 | 0 | AAD |
| 2 | 71.2574198 | 3219 | 0.53 | AAD |
| 3 | 11.8277482 | 1984 | 0 | AAD |
| 4 | 44.5050741 | 3244 | 0 | AAD |
| 5 | 147.15864 | 2233 | 0.01 | AAD |
| 6 | 66.816279 | 3041 | 0.05 | AAD |
| 7 | 136.528128 | 2507 | 0.12 | AAD |
| 8 | 153.020456 | 2999 | 0.03 | AAD |
| 9 | 159.434898 | 2561 | 0.1 | AAD |
| 10 | 162.758493 | 1099 | 10.96 | AAD |
| 11 | 33.9149044 | 1135 | 0.01 | AAD |
| 12 | 44.1538923 | 1568 | 0.01 | AAD |
| 13 | 50.6696311 | 947 | 0.01 | AAD |
| 14 | 28.1809129 | 1025 | 0 | AAD |
| 15 | 75.9797134 | 606 | 0.01 | AAD |
| 16 | 38.6482247 | 3082 | 0 | AAD |
| 17 | 120.556201 | 3599 | 0.06 | AAD |
| 18 | 61.7205173 | 3051 | 0.01 | AAD |
| 19 | 126.056353 | 2210 | 0.01 | AAD |
| 20 | 31.4281178 | 1067 | 0.01 | AAD |
| 21 | 49.9216734 | 3230 | 0 | AAD |
| 22 | 31.8591489 | 2365 | 0 | AAD |
| 23 | 103.06455 | 3327 | 0.01 | AAD |
| 24 | 38.5546901 | 500 | 0.35 | AAD |
| 25 | 78.1765625 | 1152 | 0.51 | AAD |
| 26 | 24.537785 | 3168 | 0.02 | AAD |
| 27 | 18.5739162 | 3144 | 0.01 | AAD |
| 28 | 135.095946 | 2786 | 0.35 | AAD |
| 29 | 47.3003274 | 3220 | 0.01 | AAD |
| 30 | 89.1315314 | 1760 | 0.01 | AAD |
| 31 | 96.7264371 | 1979 | 0 | AAD |
| 32 | 14.711644 | 1028 | 0 | AAD |
| 33 | 58.0426784 | 3081 | 0 | AAD |
| 34 | 98.1036596 | 3328 | 4.29 | AAD |
| 35 | 36.3854394 | 443 | 0.01 | AAD |
| 36 | 105.445272 | 2472 | 0.04 | AAD |
| 37 | 58.275625 | 2009 | 0.02 | AAD |
| 38 | 79.9837983 | 1037 | 0.21 | AAD |
| 39 | 43.6567732 | 1981 | 0.01 | AAD |
| 40 | 58.4507198 | 837 | 0.05 | AAD |
| 41 | 98.5376265 | 1659 | 0.09 | AAD |
| 42 | 49.9216734 | 624 | 0.03 | AAD |
| 43 | 34.7602901 | 1032 | 0 | AAD |
| 44 | 54.0586395 | 2170 | 0.01 | AAD |
| 45 | 150.724963 | 837 | 0.48 | AAD |
| 46 | 137.009145 | 3169 | 0.01 | AAD |
| 47 | 16.3917305 | 68 | 0.03 | AAD |
| 48 | 131.664648 | 1832 | 0.21 | AAD |
| 49 | 85.9369719 | 3409 | 0.03 | AAD |
| 50 | 39.8309546 | 3291 | 0 | AAD |
| 51 | 37.3012633 | 417 | 0.01 | AAD |
| 52 | 22.673578 | 1777 | 0.37 | AMI |
| 53 | 17.6252154 | 49 | 0.6 | AMI |
| 54 | 50.7279831 | 63 | 0.08 | AMI |
| 55 | 26.528545 | 529 | 0.64 | AMI |
| 56 | 70.6448944 | 959 | 0.01 | AAD |
| 57 | 56.1822426 | 1282 | 0.01 | AAD |
| 58 | 23.2512254 | 3412 | 0 | AAD |
| 59 | 14.2237906 | 76 | 0.37 | AMI |
| 60 | 18.3895797 | 123 | 17.7 | AMI |
| 61 | 8.21382615 | 7166 | 0.31 | APE |
| 62 | 14.884947 | 94 | 0.09 | AMI |
| 63 | 26.1111789 | 126 | 0.62 | AMI |
| 64 | 6.7473976 | 900 | 0.01 | APE |
| 65 | 15.6596533 | 57 | 0.11 | AMI |
| 66 | 16.1400673 | 1899 | 0 | AAD |
| 67 | 28.526702 | 1829 | 0 | AAD |
| 68 | 22.1926546 | 1820 | 0.04 | APE |
| 69 | 153.714914 | 98 | 0.03 | AAD |
| 70 | 25.7806805 | 1062 | 0.05 | AAD |
| 71 | 28.8233607 | 168 | 0.77 | AMI |
| 72 | 17.7281776 | 252 | 0.35 | AMI |
| 73 | 10.1601644 | 2318 | 0.01 | APE |
| 74 | 55.6532874 | 216 | 0 | AAD |
| 75 | 210.556547 | 778 | 0 | AAD |
| 76 | 26.5939638 | 105 | 0.47 | AMI |
| 77 | 8.16383043 | 1117 | 0.01 | APE |
| 78 | 12.3024385 | 181 | 0.34 | AMI |
| 79 | 5.58680841 | 2896 | 0.25 | APE |
| 80 | 4.59742635 | 44 | 2.8 | AMI |
| 81 | 5.53462797 | 135 | 0.07 | AMI |
| 82 | 5.70902094 | 1387 | 0.08 | AMI |
| 83 | 6.26042536 | 3462 | 0 | AAD |
| 84 | 22.3453239 | 126 | 0.35 | AMI |
| 85 | 14.3165257 | 64 | 0.75 | AMI |
| 86 | 8.91253073 | 126 | 0.21 | AMI |
| 87 | 36.1991639 | 61 | 0.3 | AMI |
| 88 | 112.762788 | 2836 | 0.03 | AAD |
| 89 | 13.6332746 | 99 | 0.04 | AMI |
| 90 | 32.6312378 | 226 | 40.81 | AMI |
| 91 | 17.888459 | 36 | 0.01 | AMI |
| 92 | 34.7355295 | 513 | 0 | AAD |
| 93 | 23.4930265 | 260 | 4.19 | AMI |
| 94 | 29.3746863 | 41 | 0.59 | AMI |
| 95 | 14.2830664 | 1189 | 0.09 | APE |
| 96 | 12.3346851 | 937 | 0.05 | APE |
| 97 | 14.009535 | 101 | 0.01 | AMI |
| 98 | 23.3170894 | 109 | 0.07 | AMI |
| 99 | 46.4304164 | 468 | 0 | AAD |
| 100 | 127.885339 | 1038 | 0.06 | AAD |
| 101 | 11.640314 | 77 | 0.28 | AMI |
| 102 | 9.9414733 | 76 | 0.01 | AMI |
| 103 | 46.247955 | 67 | 1.97 | AMI |
| 104 | 46.2823003 | 2713 | 0.12 | AAD |
| 105 | 10.7919832 | 131 | 0.21 | AMI |
| 106 | 34.1066792 | 57 | 0.68 | AMI |
| 107 | 9.93884908 | 425 | 1.21 | AMI |
| 108 | 21.3734634 | 2958 | 0.98 | APE |
| 109 | 12.3873205 | 365 | 0.03 | AMI |
| 110 | 5.21651666 | 1259 | 0.02 | APE |
| 111 | 36.1253807 | 1745 | 0.01 | AAD |
| 112 | 59.4563938 | 820 | 0.02 | AAD |
| 113 | 28.1883007 | 2142 | 0.01 | AAD |
| 114 | 31.0072788 | 95 | 6.09 | AMI |
| 115 | 16.3361884 | 85 | 0.05 | AMI |
| 116 | 15.2004558 | 258 | 0.19 | AMI |
| 117 | 6.84242276 | 293 | 0.85 | AMI |
| 118 | 139.250902 | 1987 | 0.05 | AAD |
| 119 | 203.641787 | 191 | 0.04 | AAD |
| 120 | 76.2972283 | 2327 | 0.01 | AAD |
| 121 | 23.4549768 | 94 | 0.01 | AMI |
| 122 | 15.5518068 | 57 | 0.34 | AMI |
| 123 | 5.83357151 | 799 | 0 | APE |
| 124 | 9.81206512 | 37 | 0.94 | AMI |
| 125 | 15.2509874 | 55 | 1.04 | AMI |
| 126 | 14.5206321 | 40 | 1.95 | AMI |
| 127 | 10.7534611 | 102 | 102 | AMI |
| 128 | 16.1571995 | 138 | 2.49 | AMI |
| 129 | 28.6969637 | 79 | 2.45 | AMI |
| 130 | 11.2434075 | 114 | 0 | AMI |
| 131 | 9.59034769 | 162 | 1.37 | AMI |
| 132 | 46.9201013 | 160 | 10.54 | AMI |
| 133 | 64.2310903 | 106 | 12.84 | AMI |
| 134 | 41.4447243 | 40 | 0.88 | AMI |
| 135 | 26.8370264 | 56 | 0.09 | AMI |
| 136 | 19.1271441 | 2226 | 0.02 | APE |
| 137 | 27.461448 | 537 | 0 | AAD |
| 138 | 142.850527 | 1126 | 0.01 | AAD |
| 139 | 15.8804698 | 106 | 0.03 | AMI |
| 140 | 23.9982581 | 646 | 0.82 | AAD |
| 141 | 13.7968962 | 43 | 2.31 | AMI |
| 142 | 12.0769825 | 51 | 2.16 | AMI |
| 143 | 12.1735492 | 65 | 0.09 | AMI |
| 144 | 181.33494 | 1142 | 0 | AAD |
| 145 | 21.5442241 | 82 | 49.44 | AMI |
| 146 | 25.3161942 | 197 | 95 | AMI |
| 147 | 113.152006 | 1171 | 0 | AAD |
| 148 | 29.965132 | 1064 | 2.4 | AAD |
| 149 | 30.5046536 | 755 | 8.62 | AAD |
| 150 | 7.44136357 | 964 | 0 | APE |
| 151 | 5.52084863 | 29 | 5.86 | AMI |
| 152 | 132.88026 | 940 | 8.87 | AAD |
| 153 | 267.299521 | 2663 | 2.31 | AAD |
| 154 | 13.6332746 | 67 | 95 | AMI |
| 155 | 177.034102 | 2825 | 1.28 | AAD |
| 156 | 56.9826006 | 1203 | 6.29 | AAD |
| 157 | 35.1842544 | 424 | 0 | AAD |
| 158 | 17.1779997 | 127 | 1.42 | AMI |
| 159 | 6.35968184 | 61 | 1.93 | AMI |
| 160 | 107.959852 | 898 | 0 | AAD |
| 161 | 13.2418142 | 28 | 0.65 | AMI |
| 162 | 10.6057554 | 53 | 23.82 | AMI |
| 163 | 18.3891606 | 126 | 7.23 | AMI |
| 164 | 28.4591766 | 47 | 4.06 | AMI |
| 165 | 2123.04927 | 2397 | 0.07 | AAD |
| 166 | 231.067764 | 3019 | 3.76 | AAD |
| 167 | 8.85956117 | 60 | 1.43 | AMI |
| 168 | 8.70451481 | 48 | 0.81 | AMI |
| 169 | 68.7723167 | 2892 | 14.05 | AAD |
| 170 | 213.41559 | 1186 | 4.04 | AAD |
| 171 | 7.89159191 | 89 | 0 | AMI |
| 172 | 301.127705 | 2344 | 0.58 | AAD |
| 173 | 127.28374 | 963 | 0.21 | AAD |
| 174 | 124.484545 | 295 | 1.24 | AAD |
| 175 | 205.140867 | 1221 | 0.12 | AAD |
| 176 | 7.40955815 | 11 | 5.04 | AMI |
| 177 | 275.397875 | 2977 | 0.05 | AAD |
| 178 | 62.7979097 | 2559 | 0.01 | AAD |
| 179 | 99.5738634 | 1566 | 0.02 | AAD |
| 180 | 148.270735 | 1013 | 4.13 | AAD |
| 181 | 145.641536 | 1006 | 0.01 | AAD |
| 182 | 2275.91016 | 236 | 0.02 | AAD |
| 183 | 134.892303 | 926 | 0 | AAD |
| 184 | 18.0740115 | 1992 | 0.01 | AAD |
| 185 | 61.5311332 | 3111 | 4.2 | AAD |
| 186 | 410.0064 | 457 | 1.05 | AAD |
| 187 | 43.348284 | 671 | 0.31 | AAD |
| 188 | 73.6750206 | 747 | 0 | AAD |
| 189 | 80.5042 | 391 | 4.1 | AAD |
| 190 | 47.4138043 | 942 | 0.01 | AAD |
| 191 | 219.615911 | 1045 | 13.57 | AAD |
| 192 | 167.298938 | 1618 | 22.38 | AAD |
| 193 | 39.7703115 | 1007 | 7.35 | AAD |
| 194 | 51.7682754 | 952 | 0 | AAD |
| 195 | 180.071571 | 1375 | 0.04 | AAD |
| 196 | 155.638763 | 2165 | 0.32 | AAD |
| 197 | 133.763776 | 3290 | 0.09 | AAD |
| 198 | 202.175092 | 2640 | 0.01 | AAD |
| 199 | 188.517775 | 1063 | 0.67 | AAD |
| 200 | 208.350939 | 1124 | 102 | AAD |
| 201 | 123.269025 | 3075 | 5.93 | AAD |
| 202 | 37.7168 | 679 | 0 | AAD |
| 203 | 42.4146233 | 1947 | 1.15 | AAD |
| 204 | 17.6792687 | 2655 | 0.19 | APE |
| 205 | 7.4910532 | 1594 | 0.36 | APE |
| 206 | 10.73307 | 239 | 0.04 | APE |
| 207 | 28.9077975 | 1583 | 0.25 | APE |
| 208 | 26.2716168 | 5878 | 0.02 | APE |
| 209 | 11.4352706 | 417 | 0 | APE |
| 210 | 203.390836 | 2119 | 0.04 | APE |
| 211 | 17.6252154 | 618 | 1.35 | APE |
| 212 | 26.9589809 | 1583 | 0.05 | APE |
| 213 | 16.4279309 | 1139 | 0 | APE |
| 214 | 7.36686274 | 1074 | 0.03 | APE |
| 215 | 7.44292572 | 454 | 0 | APE |
| 216 | 5.23872438 | 1251 | 0.13 | APE |
| 217 | 9.30752353 | 2733 | 0.07 | APE |

In summary, by rapid detection of the content of the three biomarkers - sST2, D-dimer and cardiac troponin, the levels of the three markers were conjointly analyzed: when the content of sST2 was ≥ 34.6 ng/mL, it assists in identifying the chest pain as acute aortic dissection; when content of cTNI was ≥ 0.04 ng/mL, it assists in identifying the chest pain as acute myocardial infarction; when the content of sST2 was <34.6 ng/mL and the content of D-Dimer was ≥500 ng/mL, it assists in identifying the chest pain as acute pulmonary embolism; and when the content of sST2 was < 34.6 ng/mL, the content of D-Dimer was < 500 ng/mL, and the content of cTNI was < 0.04 ng/mL, it assists in identifying the chest pain as chest pain other than the above three types of acute fatal chest pain. By improving the ability of differential diagnosis for patients with acute fatal chest pain, the risks of patients were accurately stratified, in order to increase the survival rate of patients in clinical practice.

The above provides a detailed description of the present invention. For a person skilled in the art, the present invention may be implemented within a wide range under the same parameters, concentrations and conditions without departing from the purpose and scope of the present invention and without unnecessary experiments. Although special examples are given in the present invention, it should be understood that further improvements may be made to the present invention. In summary, according to the principles of the present invention, the present application is intended to include any change, use or improvement of the present invention, including changes that deviate from the scope disclosed in the present application and are made by conventional technologies known in the art.

### Cross-reference to Related Application

This application claims priority to Chinese Patent Application No. 202410062876.2, filed on January 17, 2024 to CNIPA and entitled "DEVICE FOR IDENTIFICATION OR AUXILIARY IDENTIFICATION OF ACUTE CHEST PAIN TYPE AND USE THEREOF", the disclosure of which is herein incorporated by reference in its entirety.

### Industrial Applicability

According to the present invention, the product for differential diagnosis or assisting in differential diagnosis of a patient with acute chest pain by combination of soluble ST2, D-dimer and cardiac troponin I in the serum as indicators for the differential diagnosis of acute chest pain. The differential diagnosis of a patient with acute chest pain can be performed by detecting soluble ST2, D-dimer and cardiac troponin I in the serum. The present invention can improve the ability of differential diagnosis for acute chest pain based on known biomarkers, and is used in clinical practice to precisely stratify patient risks, thereby increasing survival rates of patients.

Sequence listing
SEQ ID No. 1
MGFWILAILTILMYSTAAKFSKQSWGLENEALIVRCPRQGKPSYTVDWYYSQTNKSIPTQE
RNRVFASGQLLKFLPAAVADSGIYTCIVRSPTFNRTGYANVTIYKKQSDCNVPDYLMYSTV
SGSEKNSKIYCPTIDLYNWTAPLEWFKNCQALQGSRYRAHKSFLVIDNVMTEDAGDYTCK
FIHNENGANYSVTATRSFTVKDEQGFSLFPVIGAPAQNEIKEVEIGKNANLTCSACFGKGTQ
FLAAVLWQLNGTKITDFGEPRIQQEEGQNQSFSNGLACLDMVLRIADVKEEDLLLQYDCL
ALNLHGLRRHTVRLSRKNPSKECF.

SEQ ID No. 2
MSDIEEVVEEYEEEEQEEAAVEEEEDWREDEDEQEEAAEEDAEAEAETEETRAEEDEEEE
EAKEAEDGPMEESKPKPRSFMPNLVPPKIPDGERVDFDDIHRKRMEKDLNELQALIEAHFE
NRKKEEEELVSLKDRIERRRAERAEQQRIRNEREKERQNRLAEERARREEEENRRKAEDE
ARKKKALSNMMHFGGYIQKTERKSGKRQTEREKKKKILAERRKVLAIDHLNEDQLREKA
KELWQSIYNLEAEKFDLQEKFKQQKYEINVLRNRINDNQKVSKTRGKAKVTGRWK.

## Claims

1. A device for identifying or assisting in identifying an acute chest pain type, wherein the device comprises the following modules:
M1, a data receiving module configured to receive a content of a protein in a serum of a test subject, wherein the protein is selected from the group consisting of soluble ST2, D-dimer and cardiac troponin I, and the test subject is a patient with acute chest pain; and
M2, a result output module configured to output, from a computer, the acute chest paintype on the basis of a concentration of the protein; wherein
the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

2. The device according to claim 1, wherein the result output module comprises a model construction sub-module; and the model construction sub-module is used to construct a model by taking the concentration of the protein in the serum of a patient with acute chest pain as input data of the model and taking the acute chest pain type as output data.

3. A method for identifying or assisting in identifying an acute chest pain type, wherein the method comprises: receiving a content of a protein in a serum of a patient with acute chest pain, wherein the protein is selected from the group consisting of soluble ST2, D-dimer and cardiac troponin I; and constructing a prediction model for an acute chest pain type by taking the content of the protein as model input data and taking the acute chest pain type as output data.

4. A method for identifying or assisting in identifying an acute chest pain type, wherein the method comprises: receiving a content of a protein in the serum of a patient with acute chest pain, wherein the protein is selected from the group consisting of soluble ST2, D-dimer and cardiac troponin I; and outputting the acute chest pain type from a computer on the basis of a concentration of the protein, wherein the acute chest pain type is acute aortic dissection, acute myocardial infarction, acute pulmonary embolism or other type of acute chest pain, and the other type of acute chest pain is acute chest pain other than acute aortic dissection, acute myocardial infarction and acute myocardial infarction.

5. The method according to claim 4, wherein the method comprises: constructing a model, wherein the constructing the model comprises: receiving the content of soluble ST2, D-dimer and cardiac troponin I in the serum of a patient with acute chest pain as model input data; and constructing the prediction model for the acute chest pain type by means of using the model input data and by taking the acute chest pain type as output data.

6. A computer-readable storage medium storing a computer program, wherein the computer program causes a computer to execute the steps of the method according to any one of claims 3 to 5.

7. A product for identifying or assisting in identifying an acute chest pain type, wherein the product comprises a device and a reagent, the device is the device according to claim 1 or 2; the reagent is a protein and/or a substance for the detection of the protein; the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

8. Use of a protein in the preparation of a product for identifying or assisting in identifying an acute chest pain type, wherein the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.

9. Use of a substance for the detection of a protein in the preparation of a product for identifying or assisting in identifying an acute chest pain type, wherein the protein is selected from the group consisting of soluble ST2, D-dimer and cardiac troponin I; and the acute chest pain type is acute aortic dissection, acute myocardial infarction, acute pulmonary embolism or other type of acute chest pain, wherein the other type of acute chest pain is acute chest pain other than acute aortic dissection, acute myocardial infarction and acute myocardial infarction.
